# FASCICULE DE BREVET EUROPEEN

(11) **EP 4 455 531 B1**
(45) Date de publication et mention de la délivrance du brevet: **11.06.2025**
(21) Numéro de dépôt: 24171124.1
(22) Date de dépôt: 18.04.2024
(51) Int. Cl.: F16L 25/00, A61M 39/18, B29C 65/00, F16L 29/00

(54) **DISPOSITIF DE RACCORDEMENT ASEPTIQUE D'UN TUBE**
VORRICHTUNG ZUM ASEPTISCHEN VERBINDEN EINES ROHRES
DEVICE FOR ASEPTIC CONNECTION OF A TUBE

(30) Priorité: 28.04.2023 FR 2304361
(43) Date de publication de la demande: 30.10.2024
(73) Titulaire: Parker Hannifin EMEA S.à.r.l., 1163 Etoy (CH)
(72) Inventeur: GUILLARD, Philippe, 35760 Saint-Gregoire (FR)
(74) Mandataire: Cabinet Boettcher

(56) Documents cités:
- FR-A1- 3 117 569
- US-A1- 2018 296 817
- US-A1- 2023 122 306

## Description

La présente invention concerne le domaine du transport de fluides liquides ou gazeux et plus particulièrement un dispositif de raccordement aseptique destiné à réaliser le raccordement de deux tubes stériles.

### ARRIERE PLAN DE L'INVENTION

On connaît des ensembles de raccordement destinés à connecter des canalisations stériles reliées à des éléments de circuits de l'industrie pharmaceutiques, tels que des filtres, des centrifugeuses, des pompes, des réservoirs en plastique souple ou des conteneurs de traitement comme des bio-processeurs.

Un tel ensemble comprend généralement deux dispositifs de raccordement destinés à être connectés entre eux après que chacun ait été raccordé à une extrémité de canalisation. Chaque dispositif comprend un corps tubulaire délimitant un canal agencé pour recevoir l'extrémité de canalisation. Le corps comporte une face frontale sur laquelle est fermement fixé un élément d'étanchéité annulaire avec lequel l'extrémité de canalisation est destinée à rentrer en contact étanche. L'élément d'étanchéité est recouvert d'un film de protection en forme de bande repliée sur elle-même dont une partie adhère de manière amovible à un flanc latéral libre de l'élément d'étanchéité et une autre partie forme une languette de tirage s'étendant en saillie de la première partie.

Après que les extrémités de canalisation aient été emmanchées dans les corps, les dispositifs sont mis coaxialement en regard puis rapprochés l'un de l'autre jusqu'à mettre en contact les films de protection et comprimer légèrement les éléments d'étanchéité. Le retrait simultané des films de protection par tirage des languettes entraîne une mise en contact étanche des flancs latéraux des éléments d'étanchéité entre eux et donc la formation d'un canal aseptique de passage de fluide entre les deux canalisations. Un rapprochement supplémentaire des dispositifs est réalisé avant de verrouiller les positions relatives des dispositifs au moyen d'une bride de serrage.

A la fin d'un processus de fabrication d'un lot de produits pharmaceutiques, l'ensemble des canalisations et des dispositifs de raccordement sont généralement démontés et incinérés. Leur reconditionnement (lavage, stérilisation...) s'avère en effet coûteux et présente un risque de dégradation des dispositifs (usure, rupture ou bien encore perte d'un composant).

Néanmoins, il apparaît que de tels dispositifs comportent de nombreux composants les rendant particulièrement coûteux pour un usage unique. Il serait par ailleurs souhaitable de limiter voire supprimer l'écoulement du fluide resté prisonnier dans les canalisations lors de la déconnexion des dispositifs.

Pour cela, il a été envisagé dans le document FR-A-3117569 d'équiper les dispositifs de raccordement d'un élément d'étanchéité permettant à la fois d'assurer l'étanchéité du raccordement du tube et d'obstruer totalement le canal avant la déconnexion. Il s'avère toutefois possible, avec de tels dispositifs, de procéder à leur déconnexion sans avoir préalablement procéder à l'obturation des canaux par les éléments d'étanchéité, de sorte qu'une erreur humaine est possible.

### OBJET DE L'INVENTION

L'invention a donc pour but de proposer un dispositif de raccordement aseptique d'un tube permettant d'obvier au moins en partie aux problèmes précités.

### RESUME DE L'INVENTION

A cet effet, l'invention propose un dispositif de raccordement aseptique d'un tube comprenant :
- un insert tubulaire délimitant un canal et ayant un premier tronçon d'extrémité pourvu de moyens de sa connexion au tube et un deuxième tronçon d'extrémité qui est destiné à s'étendre à l'extérieur du tube ;
- un corps annulaire comprenant un premier tronçon d'extrémité monté sur le deuxième tronçon d'extrémité de l'insert et un deuxième tronçon d'extrémité s'étendant axialement en saillie du deuxième tronçon d'extrémité de l'insert pour définir avec celui-ci un logement ;
- un élément d'étanchéité annulaire qui est disposé dans le logement entre une face frontale du deuxième tronçon d'extrémité de l'insert et un redan interne du deuxième tronçon du corps annulaire et qui est déformable depuis un premier état dans lequel l'élément d'étanchéité délimite une section de passage de fluide et un deuxième état dans lequel l'élément d'étanchéité obstrue totalement le canal, l'élément d'étanchéité étant agencé pour être amené de son premier état vers son deuxième état sous l'effet d'une compression axiale ; et
- des moyens de couplage à un autre dispositif de raccordement.

Le premier tronçon d'extrémité du corps est monté sur l'insert pour être déplaçable axialement entre une position en saillie axiale de l'insert dans laquelle l'élément d'étanchéité est dans son premier état et une position affleurante de l'insert dans laquelle l'élément d'étanchéité est suffisamment comprimé axialement pour être dans son deuxième état.

Selon l'invention :
- un manchon de manœuvre est monté mobile en rotation sur le corps entre une position de connexion et une position de déconnexion, et est couplé à l'insert pour déplacer axialement le corps de la position en saillie à la position affleurante en réponse à une rotation du manchon de la position de connexion à la position de déconnexion ; et
- les moyens de couplage sont agencés pour permettre un découplage du dispositif de raccordement et de l'autre dispositif de raccordement uniquement lorsque le manchon est en position de déconnexion.

Ainsi, il est nécessaire d'amener le manchon de manœuvre en position de déconnexion, et donc de procéder à l'obturation du canal par l'élément d'étanchéité pour désaccoupler les dispositifs l'un de l'autre, de sorte que tout risque d'erreur humaine est limité.

De manière particulière, le dispositif comprend des moyens de verrouillage du corps en position affleurante.

De manière particulière, l'élément d'étanchéité comprend une face frontale en saillie du deuxième tronçon du corps annulaire et sur laquelle est fixé de manière amovible un film de protection sur lequel est rabattue une languette de tirage ayant une première extrémité reliée à un bord du film de protection et une deuxième extrémité s'étendant en saillie d'un bord du film opposé à la première extrémité de la languette de tirage.

Ainsi, l'élément d'étanchéité permet à la fois d'assurer l'étanchéité du raccordement du tube et d'obstruer totalement le canal, et donc de limiter le nombre de composants du dispositif.

De manière particulière, le premier tronçon d'extrémité du corps comprend une patte de verrouillage s'étendant en saillie d'une face frontale du corps et comportant à une extrémité libre une aspérité destinée à coopérer avec un bord de l'autre dispositif de raccordement, et une extrémité du manchon est pourvue extérieurement d'au moins une portion de collet destinée à coopérer avec une patte de verrouillage de l'autre dispositif de raccordement, la portion de collet et la patte de verrouillage formant les moyens de verrouillage.

De manière particulière, l'insert tubulaire est pourvu d'une tige s'étendant axialement en porte-à-faux dans le canal pour avoir une portion d'extrémité libre en saillie du deuxième tronçon d'extrémité de l'insert. La section de passage de fluide délimité par l'élément d'étanchéité dans son premier est alors supérieure à une section transversale de la tige et l'élément d'étanchéité dans son deuxième état enserre la tige.

De manière particulière, la rotation du manchon depuis la position de connexion à la position de déconnexion est sensiblement égale à 180 degrés.

L'invention concerne également un ensemble de raccordement de deux tubes comprenant deux tels dispositifs de raccordement.

Avantageusement, les deux dispositifs de raccordement sont identiques.

L'invention concerne aussi un procédé de déconnexion de deux tubes raccordés à l'aide d'un tel ensemble de raccordement, le procédé comprenant les étapes suivantes :
- amener les manchons des deux dispositifs de la position de connexion à la position de déconnexion ; et
- éloigner axialement les dispositifs l'un de l'autre.

### BREVE DESCRIPTION DES DESSINS

L'invention sera mieux comprise à la lumière de la description qui suit, laquelle est purement illustrative et non limitative, et doit être lue en regard des dessins annexés, parmi lesquels :
[Fig. 1] la figure 1 est une vue en perspective d'un dispositif de raccordement selon un mode de réalisation particulier de l'invention ;
[Fig. 2] la figure 2 est une vue analogue à la figure 1, dans laquelle le dispositif de raccordement est dépourvu de son capot de protection. ;
[Fig. 3] la figure 3 est une vue éclatée du dispositif de raccordement illustré à la figure 2 ;
[Fig. 4] la figure 4 est une vue en coupe transversale du dispositif de raccordement illustré à la figure 2 ;
[Fig. 5] la figure 5 est une vue en coupe axiale du dispositif de raccordement illustré à la figure 2 ;
[Fig. 6A] la figure 6A est une vue en perspective d'un ensemble de raccordement aseptique selon un mode de réalisation particulier de l'invention, avant connexion ;
[Fig. 6B] la figure 6B est une vue en coupe transversale de l'ensemble de raccordement illustré à la figure 6A ;
[Fig. 7A] la figure 7A est une vue analogue à la figure 6A, après la connexion et avant le retrait du film de protection ;
[Fig. 7B] la figure 7B est une vue en coupe transversale de l'ensemble de raccordement illustré à la figure 7A ;
[Fig. 8A] la figure 8A est une vue analogue à la figure 6A, après la connexion et après le retrait du film de protection ;
[Fig. 8B] la figure 8B est une vue en coupe transversale de l'ensemble de raccordement illustré à la figure 8A ;
[Fig. 9A] la figure 9A est une vue analogue à la figure 6A, pendant la déconnexion ;
[Fig. 9B] la figure 9B est une vue en coupe transversale de l'ensemble de raccordement illustré à la figure 9A ;
[Fig. 10A] la figure 10A est une vue analogue à la figure 6A, après la déconnexion ;
[Fig. 10B] la figure 10B est une vue en coupe transversale de l'ensemble de raccordement illustré à la figure 10A ;
[Fig. 11] la figure 11 est une vue en coupe axiale d'une variante du dispositif de raccordement illustré à la figure 1, dépourvue de son capot de protection.

### DESCRIPTION DETAILLEE DE L'INVENTION

L'invention est ici décrite en application au raccordement d'un premier tube 100 stérile formant un premier conduit de transport de fluide à un deuxième conduit de transport de fluide formé par un deuxième tube 200 stérile.

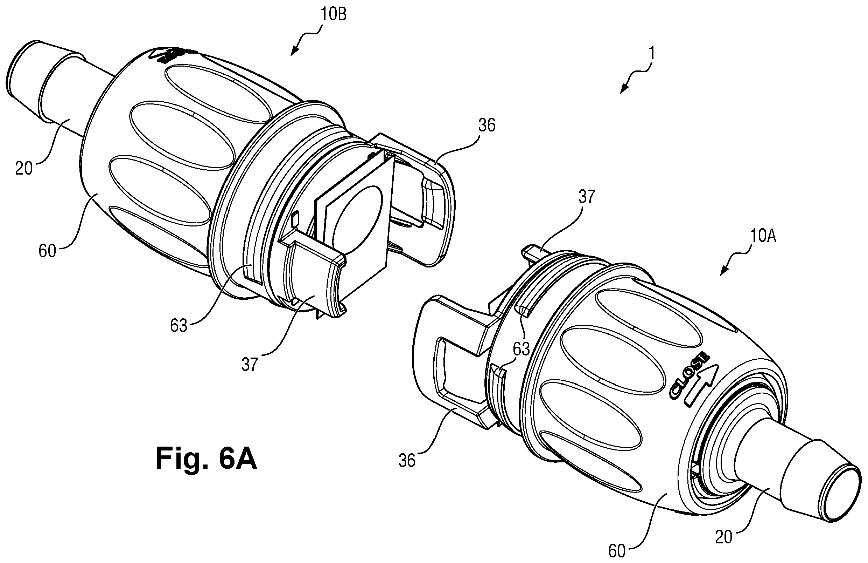

En référence aux figures 6A à 10B, un ensemble de raccordement fluidique généralement désigné en 1, selon un mode de réalisation particulier de l'invention, comprend deux dispositifs 10 de raccordement destinés à réaliser ensemble une connexion aseptique entre le premier tube 100 et le deuxième tube 200. Les deux dispositifs 10 sont identiques et sont distingués sur les figures par l'adjonction des lettres A, B.

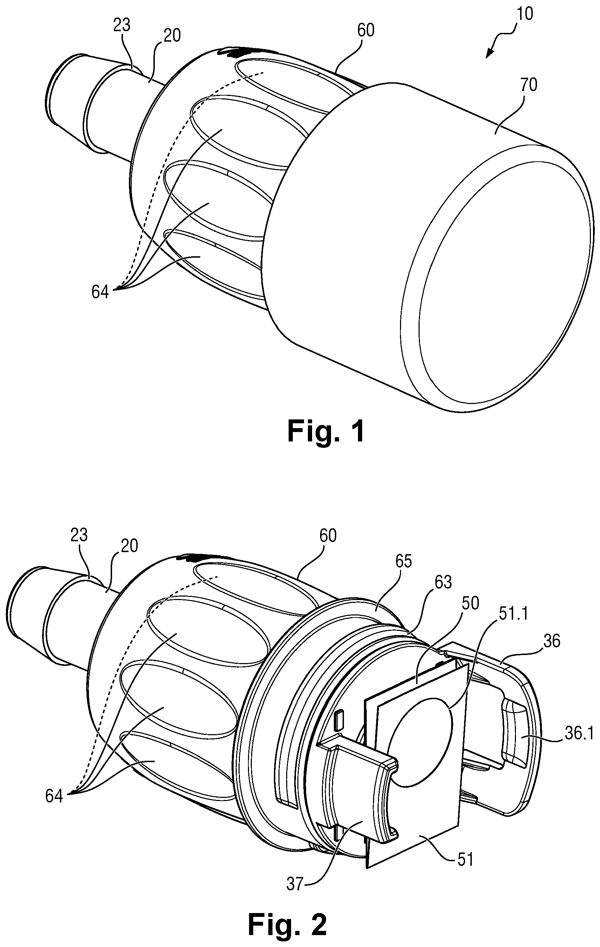

Comme illustré aux figures 1 à 5, chaque dispositif 10 comprend un embout ou insert 20 tubulaire délimitant un canal 21 de passage de fluide.

L'insert 20 comporte un premier tronçon 20.1 d'extrémité délimitant un premier alésage 22.1 formant une première portion du canal 21. Le premier tronçon 20.1 comprend extérieurement un relief 23 en dent de sapin et une collerette 24 formant respectivement un moyen de son ancrage dans le tube 100, 200 et une butée à son enfoncement dans ledit tube 100, 200.

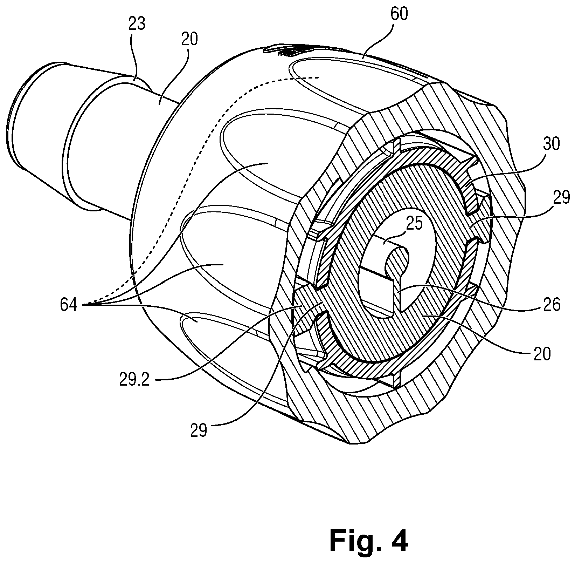

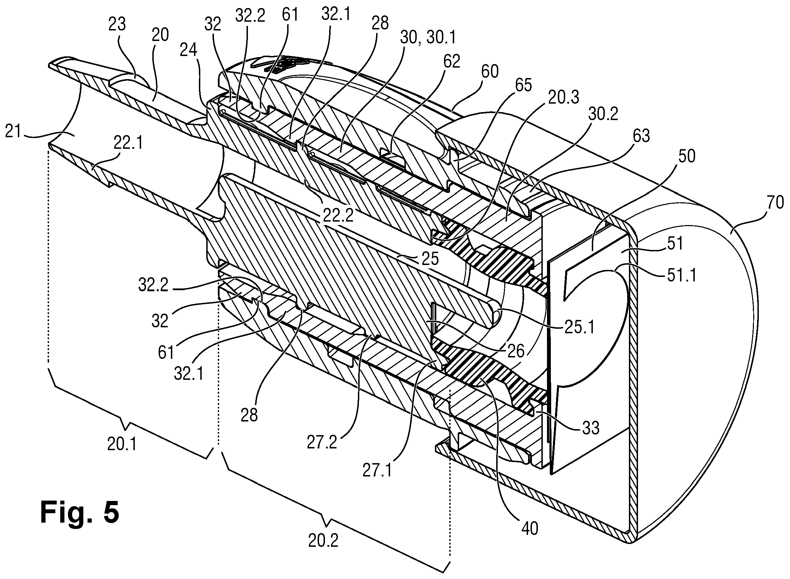

L'insert 20 comporte un deuxième tronçon 20.2 d'extrémité délimitant un deuxième alésage 22.2 coaxial au premier alésage 22.1 et formant une deuxième portion du canal 21. Le deuxième tronçon 20.2 est pourvu intérieurement d'une tige 25 de forme cylindrique s'étendant axialement en porte à faux dans le canal 21 de sorte qu'une portion d'extrémité libre 25.1 de la tige s'étend en saillie du deuxième tronçon 20.2 de l'insert 20. Comme illustré aux figures 4 et 5, la tige 25 est reliée axialement au deuxième alésage 22.2 par un voile 26 s'étendant axialement. Le diamètre de la tige 25 est ici suffisamment faible pour que la section de passage de fluide du canal 21 soit ici quasi identique dans le premier tronçon 20.1 et le deuxième tronçon 20.2.

Le deuxième tronçon 20.2 de l'insert 20 est pourvu extérieurement de deux collerettes 27.1, 27.2 coaxiales agencées à une extrémité libre dudit deuxième tronçon 20.2, et d'un anneau de crantage 28 ayant un diamètre extérieur légèrement supérieur à celui des collerettes 27.1, 27.2. Les collerettes 27.1, 27.2 forment des premiers moyens de centrage d'un corps 30 tubulaire monté sur l'insert 20. L'anneau de crantage 28 forme un deuxième moyen de centrage du corps 30 sur l'insert 20 et, comme on le verra plus loin, un moyen de verrouillage axial du corps 30 en position sur l'insert 20.

Le deuxième tronçon 20.2 de l'insert 20 est également pourvu extérieurement de deux nervures 29 axiales diamétralement opposées et agencées, comme on le verra plus loin, pour lier en rotation le corps 30 à l'insert 20. Les nervures 29 comportent des faces latérales comprenant des évidements agencés pour former des flancs d'arrêt 29.1 du corps sur l'insert 20.

Le corps 30 comprend un premier tronçon 30.1 d'extrémité monté sur le deuxième tronçon 20.2 de l'insert 20, et un deuxième tronçon 30.2 d'extrémité s'étendant axialement en saillie dudit deuxième tronçon 20.2 de l'insert 20.

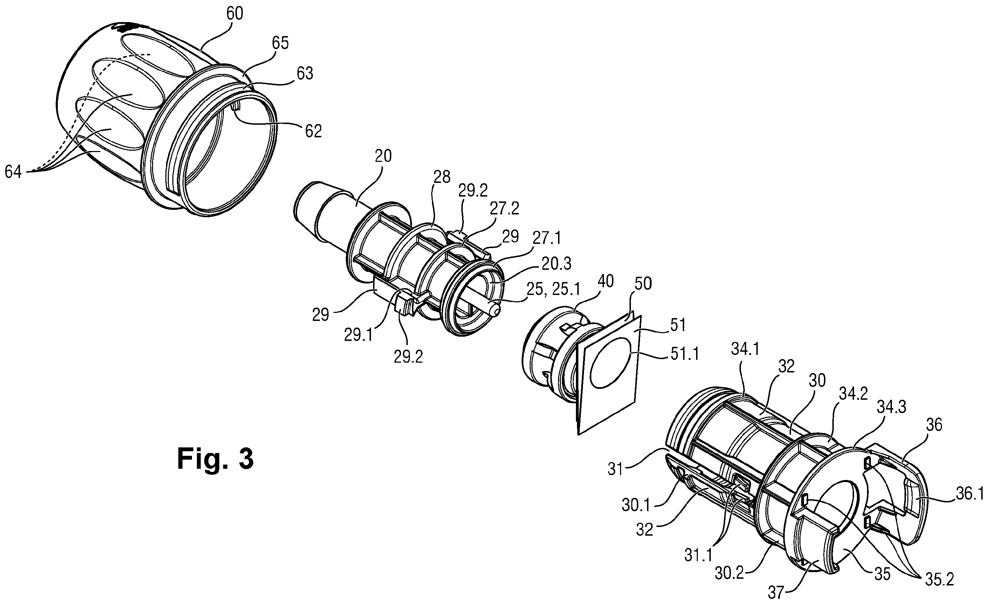

Comme illustré à la figure 3, le premier tronçon 30.1 comporte deux rainures 31 axiales débouchant sur un bord libre du premier tronçon 30.1 pour délimiter entre elles deux portions cylindriques 32 diamétralement opposées. Les portions cylindriques 32 ont chacune une extrémité solidaire du reste du premier tronçon 30.1 et une extrémité opposée comprenant un relief 32.1 interne coopérant avec un flanc latéral de l'anneau de crantage 28, et une gorge 32.2 annulaire externe agencée, comme on le verra plus loin, pour lier en translation le corps 30 à un manchon 60 de manœuvre monté sur ledit corps 30. Les rainures 31 délimitant les portions cylindriques 32 sont agencées pour recevoir les nervures 29 de l'insert 20 de manière à lier en rotation le corps 30 à l'insert 20 et à assurer un déplacement axial du corps 30 sur l'insert 20. Les portions cylindriques 32 sont élastiquement déformables entre un état de repos dans lequel les reliefs 32.1 délimitent une section de passage inférieure au diamètre de l'anneau de crantage 28 et un premier état déformé dans lequel lesdits reliefs 32.1 délimitent une section de passage supérieure au diamètre dudit anneau de crantage 28. On notera que l'anneau de crantage 28 a un pourtour extérieur comportant un chanfrein agencé pour ne pas gêner le montage du corps 30 sur l'insert 20.

Les rainures 31 délimitant les portions cylindriques 32 comprennent également un tronçon délimité par deux bras 31.1 élastiquement déformables entre un état de repos dans lequel les bras 31.1 délimitent une section de passage inférieure à une largeur des nervures 29 de l'insert 20, et un état déformé dans lequel lesdites bras 31.1 délimitent une section de passage supérieure à la largeur desdites nervures 29.

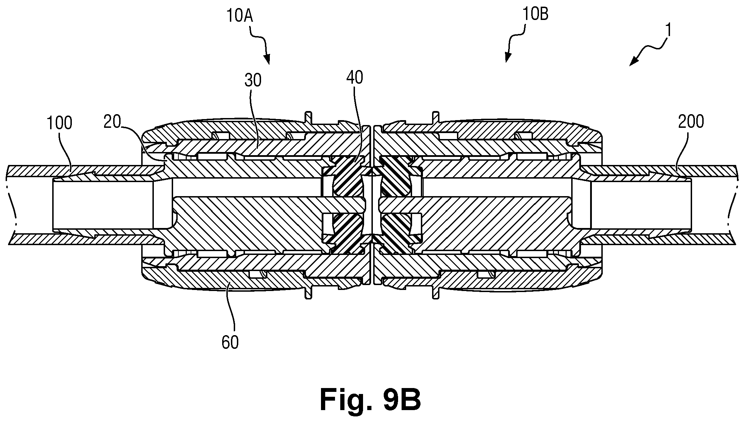

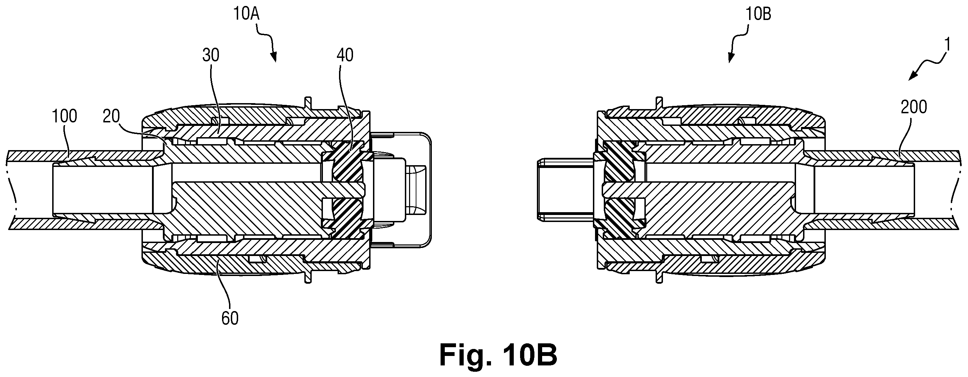

Les nervures 29 comportent une face avant agencée pour ne pas globalement s'opposer à un déplacement axial du corps 30 depuis une position en saillie, dans laquelle les reliefs 32.1 des portions cylindriques 32 coopèrent avec le flanc latéral de l'anneau de crantage 28 et dans laquelle les bras 31.1 sont éloignés axialement des nervures 29 (figure 5, 6B, 7B, 8B), vers une position affleurant l'insert 20, dans laquelle une extrémité libre des bras 31.1 coopèrent à l'état de repos avec les flancs d'arrêt 29.1 ménagés sur des faces latérales des nervures 29 (figure 9B et 10B) de manière à s'opposer à un déplacement axial du corps 30 depuis la position affleurante vers la position en saillie. On comprend qu'après avoir rejoint la position affleurante, le corps 30 ne peut plus rejoindre la position en saillie.

Le deuxième tronçon 30.2 du corps 30 définit avec le deuxième tronçon 20.2 de l'insert 20, en regard de la portion d'extrémité 25.1 de la tige 25, un logement de réception d'un élément d'étanchéité 40 annulaire qui est disposé entre une face frontale 20.3 du deuxième tronçon 20.2 et un redan 33 annulaire interne du deuxième tronçon 30.2 agencé à une extrémité libre dudit deuxième tronçon 30.2.

Le premier tronçon 30.1 et le deuxième tronçon 30.2 du corps 30 sont chacun pourvus extérieurement d'une collerette 34.1, 34.2 formant des moyens de centrage du manchon 60 sur le corps 30. Le deuxième tronçon 30.2 est également pourvu d'une autre collerette 34.3 qui s'étend radialement dans le prolongement du redan 33 pour définir avec celui-ci une face frontale 35 du corps 30. Les collerettes 34.1, 34.2, 34.3 sont coaxiales et la face frontale 35 du corps 30 est pourvue de quatre plots 35.2 délimitant chacun une surface de contact destinés à coopérer axialement avec une surface de contact d'un plot 35.2 d'un autre dispositif 10 pour garantir, comme on le verra plus loin, un léger jeu entre les faces frontales 35 après le rapprochement des deux dispositifs 10.

Le corps 30 comprend en outre une patte de verrouillage 36 et une broche d'assemblage 37 qui sont diamétralement opposées et qui s'étendent axialement en saillie de la face frontale 35 du corps 30, au voisinage d'un pourtour de celle-ci.

La patte de verrouillage 36 est élastiquement déformable et comprend une extrémité libre ayant une surface intérieure pourvue d'une aspérité 36.1 en dent de sapin destinée à assurer, comme on le verra plus loin, un accouplement du dispositif 10 avec l'autre dispositif 10.

La face frontale 35 du corps 30 comprend une fente 35.1 destinée à recevoir une portion de la broche d'assemblage 37 de l'autre dispositif 10. La fente 35.1 est agencée au voisinage d'un pourtour de la face frontale 35, à proximité de la patte de verrouillage 36, la fente 35.1 et la broche d'assemblage 37 étant diamétralement opposées.

L'élément d'étanchéité 40 est en contact étanche avec la face frontale 20.3 du deuxième tronçon 20.2 de l'insert 20 et un flanc interne du redan 33 en regard de ladite face frontale 20.3. L'élément d'étanchéité 40 est déformable depuis un premier état dans lequel il délimite une section de passage supérieure à une section transversale de la tige 25 de l'insert 20 et un deuxième état dans lequel il enserre la tige 25, et est agencé pour être amené de son premier état vers son deuxième état sous l'effet d'une compression axiale. Un tel élément d'étanchéité est connu du document EP2569665.

Lorsque le corps 30 est déplacé axialement de la position en saillie à la position affleurante, l'insert 20 comprime suffisamment l'élément d'étanchéité 40 contre le redan 33 pour l'amener de son premier état à son deuxième état.

L'élément d'étanchéité 40 comprend une face frontale s'étendant en saillie du deuxième tronçon 30.2 du corps 30 et sur laquelle est fixé de manière amovible un film de protection 50 sur laquelle est rabattue une languette 51 de tirage permettant de retirer le film de protection 50 sans toucher à celui-ci. La languette 51 de tirage a une première extrémité reliée à un bord du film de protection 50 et une deuxième extrémité s'étendant en saillie d'un bord du film de protection 50 opposé à la première extrémité de la languette 51. La deuxième extrémité de la languette 51 comprend en son centre un trou 51.1 conformé pour permettre le passage d'un doigt à travers ledit trou 51.1 de manière à améliorer la préhension de la languette 51 en formant un anneau de traction.

Le manchon 60 comprend intérieurement un redan 61 annulaire reçu dans les gorges 32.2 externes des portions cylindriques 32 du corps 30 de sorte que le corps 30 est lié en translation au manchon 60.

Les portions cylindriques 32 sont élastiquement déformables entre leur état de repos dans lequel l'extrémité libre des portions cylindriques 32 délimite une section de passage supérieure au diamètre du redan 61, et un deuxième état déformé dans lequel ladite extrémité libre délimite une section de passage inférieure au diamètre dudit redan 61. On notera que l'extrémité libre des portions cylindriques 32 a un pourtour extérieur comportant un chanfrein agencé pour ne pas gêner le montage du manchon 60 sur le corps 30.

Le manchon 60 comprend également intérieurement une rainure 62 hélicoïdale formant un filetage agencé pour recevoir des excroissances 29.2 s'étendant radialement en saillie des nervures 29 de l'insert 20 de manière à ce qu'une rotation axiale du manchon 60 de manœuvre d'une position de connexion à une position de déconnexion entraîne un déplacement axial de l'insert 20 de la position en saillie à la position affleurante. L'angle rotation du manchon 60 depuis la position de connexion à la position de déconnexion est ici sensiblement égale à 180 degrés.

Le manchon 60 comprend une extrémité pourvue extérieurement de deux portions de collet 63, identiques et diamétralement opposées, sur lesquelles la patte de verrouillage 36 du corps 30 de l'autre dispositif 10 est destinée à venir se clipper via son aspérité 36.1 en dent de sapin pour assurer un accouplement du dispositif 10 avec ledit autre dispositif 10 dont le manchon 60 est en position de connexion. Les portions de collets 63 sont espacées angulairement de manière à délimiter un chemin de dégagement axial de la patte de verrouillage 36 de l'autre dispositif 10 lorsque le manchon 60 est en position de déconnexion.

Le manchon 60 comprend aussi extérieurement une pluralité d'évidements 64 répartis angulairement de manière régulière pour former des moyens de préhension du manchon 60 permettant de faciliter sa rotation.

Afin d'éviter toute détérioration du film de protection 50 et contamination de l'élément d'étanchéité 40 lors du stockage et du transport du dispositif 10, un capot 70 de protection est monté de manière amovible sur l'extrémité du manchon 60. Le capot 70 est ici agencé pour se clipper (par emboîtement élastique) sur un redan 65 annulaire externe du manchon 60 et est destiné à être retiré juste avant le couplage du dispositif 10 à l'autre dispositif 10.

On notera que l'ensemble du dispositif 10 est stérilisé en usine afin d'être exempt de toute contamination avant son stockage.

Le fonctionnement de l'ensemble de raccordement 1 va maintenant être détaillé en regard des figures 6A à 10B.

Le tronçon 20.1 d'extrémité du premier dispositif 10A est tout d'abord emmanché dans le premier tube 100 jusqu'à ce que celui-ci vienne en butée contre la collerette 24 dudit premier dispositif 10A. De même, le tronçon 20.1 d'extrémité du deuxième dispositif 10B est emmanché dans le deuxième tube 200 jusqu'à ce que celui-ci vienne en butée contre la collerette 24 dudit deuxième dispositif 10B, de sorte que les premier et deuxième dispositifs 10A, 10B sont respectivement ancrés dans les premier et deuxième tubes 100, 200.

Les capots 70 de protection des premier et deuxième dispositifs 10A, 10B sont alors retirés des manchons 60 rendant notamment visibles les pattes de verrouillage 36 et les broches d'assemblage 37 des corps 30.

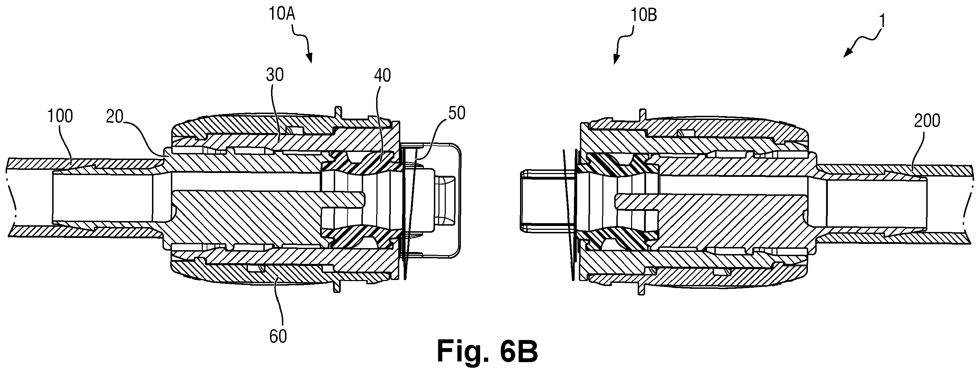

Les premier et deuxième dispositifs 10A, 10B sont ensuite présentés coaxialement en regard l'un de l'autre, le premier dispositif 10A étant tourné axialement de sensiblement 180 degrés par rapport au deuxième dispositif 10B de sorte que la broche d'assemblage 37 et la fente 35.1 du premier dispositif 10A sont respectivement en regard de la fente 35.1 et de la broche d'assemblage 37 du deuxième dispositif 10B (figures 6A et 6B).

Dans un premier temps, les premier et deuxième dispositifs 10A, 10B sont rapprochés l'un de l'autre jusqu'à ce que les languettes 51 de tirage des premier et deuxième dispositifs 10A, 10B soient en contact, la fente 35.1 du premier dispositif 10A recevant alors une portion de la broche d'assemblage 37 du deuxième dispositif 10B et la fente 35.1 du deuxième dispositif 10B recevant une portion de la broche d'assemblage 37 du premier dispositif 10A.

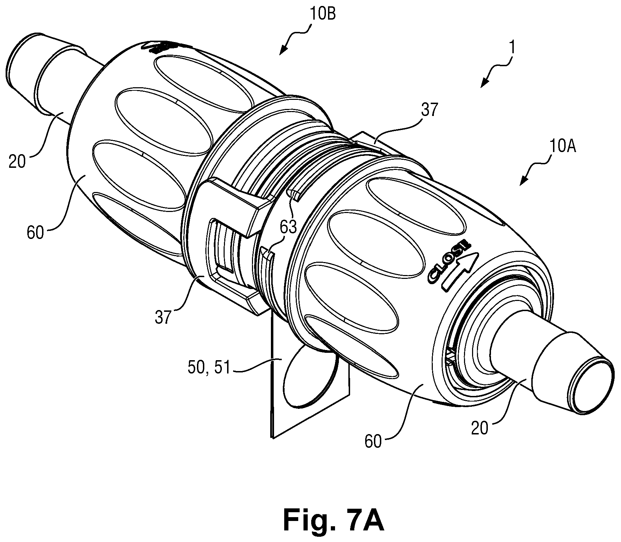

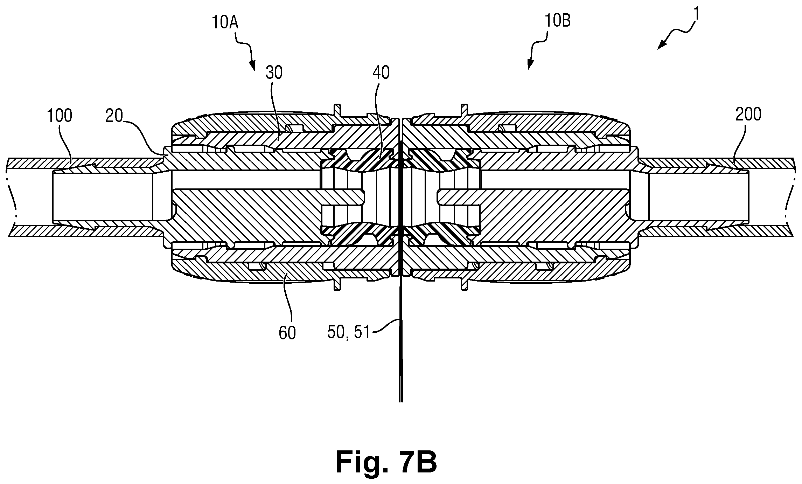

En poursuivant le rapprochement des premier et deuxième dispositifs 10A, 10B jusqu'à ce que les plots 35.2 des faces frontales 35 soient en contact les unes avec les autres, les films de protection 50 sont plaqués l'un contre l'autre, ce qui entraîne une légère compression d'une portion d'extrémité des éléments d'étanchéité 40 s'étendant en saillie de ladite face frontale, mais aussi une coopération de l'aspérité 36.1 de la patte de verrouillage 36 du premier dispositif 10A avec l'une des portions de collet 63 du deuxième dispositif 10B et une coopération de l'aspérité 36.1 de la patte de verrouillage 36 du deuxième dispositif 10B avec l'une des portions de collet 63 du premier dispositif 10A (figures 7A et 7B). On parle alors de couplage des premier et deuxième dispositifs 10A, 10B. Les plots 35.2 définissent un léger jeu entre les faces frontales 35, ce qui permet d'éviter le pincement des films de protection 50 entre lesdites faces frontales 35.

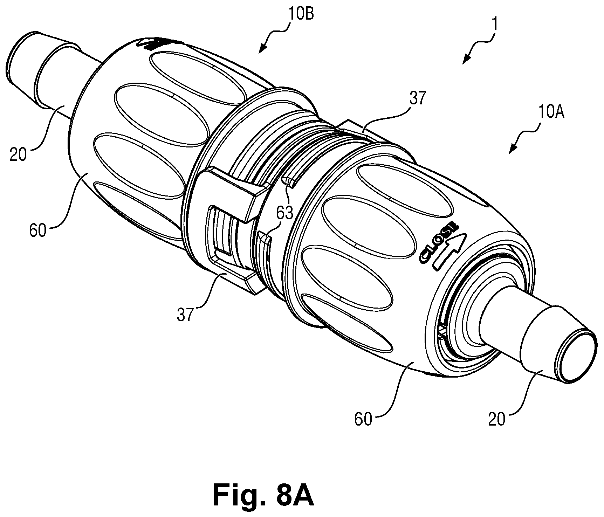

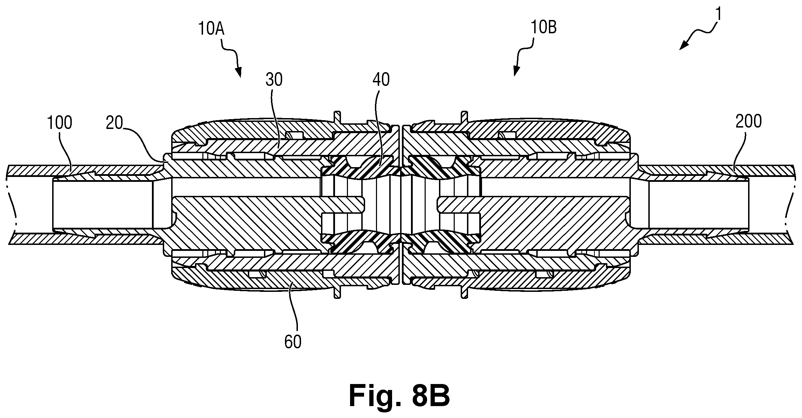

Les films de protection 50 sont alors retirés en passant un même doigt à travers les trous 51.1 des languettes 51 des premier et deuxième dispositifs 10A, 10B et en tirant simultanément sur lesdites languettes 51, provoquant alors une décompression des portions d'extrémité des éléments d'étanchéité 40 et une mise en contact étanche de celles-ci (figures 8A et 8B). Les premier et deuxième dispositifs 10A, 10B forment ainsi ensemble un canal pour le passage d'un fluide entre le premier tube 100 et le deuxième tube 200.

On comprendra que le léger jeu entre les faces frontales 35 permet de faciliter le retrait des films de protection 50.

Lorsqu'il convient de déconnecter le premier dispositif 10A du deuxième dispositif 10B, un couple C₁, C₂ d'entraînement en rotation est exercé, de préférence simultanément, sur chacun des manchons 60 de manière à amener lesdits manchons 60 de la position de connexion vers la position de déconnexion et ainsi déplacer axialement les corps 30 des premier et deuxième dispositifs 10A, 10B de la position en saillie vers la position affleurante.

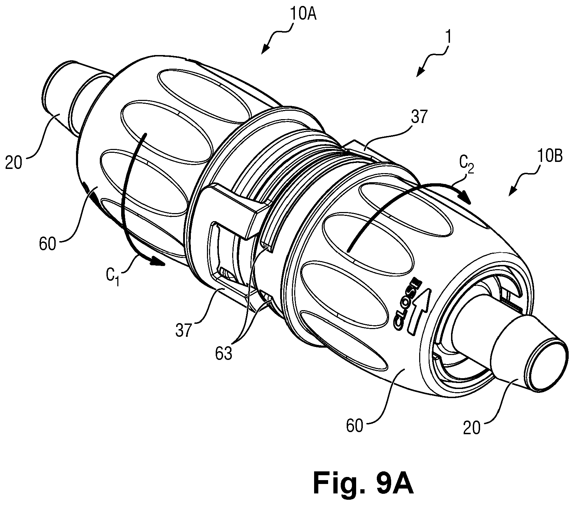

Une fois les corps 30 en position affleurante (figures 9A et 9B) :
- les bras 31.1 des rainures 31 s'opposent au déplacement axial des corps 30 vers la position en saillie sous l'effet des efforts engendrés par la compression des éléments d'étanchéités 40 ;
- les éléments d'étanchéité 40 des premier et deuxième dispositifs 10A, 10B sont suffisamment comprimés pour enserrer les tiges 25 des inserts 20 de sorte que le fluide ne peut plus circuler entre le premier tube 100 et le deuxième tube 200, et plus particulièrement entre le premier dispositif 10A et le deuxième dispositif 10B ; et
- la patte de verrouillage 36 du premier dispositif 10A est en regard du chemin de dégagement délimité par les portions de collet 63 équipant le manchon 60 du deuxième dispositif 10B, et la patte de verrouillage 36 du deuxième dispositif 10B est en regard du chemin de dégagement délimité par les portions de collet 63 équipant le manchon 60 du premier dispositif 10A, de sorte que lesdites pattes de verrouillage 36 ne s'opposent plus à la déconnexion des premier et deuxième dispositifs 10A, 10B.

Les couples C₁, C₂ d'entraînement exercés sur les manchons 60 des premier et deuxième dispositifs 10A, 10B sont alors stoppés.

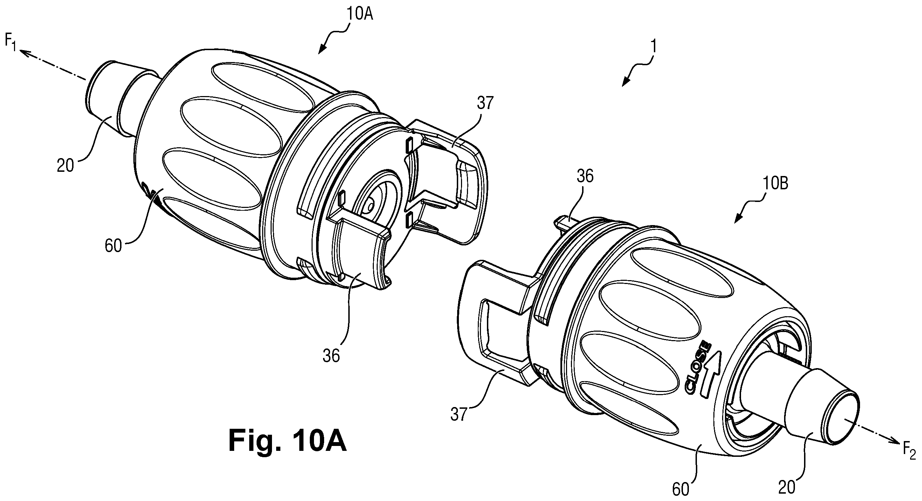

Un effort axial F₁, F₂ est ensuite exercé sur les manchons 60 de manière à désolidariser le premier dispositif 10A et le deuxième dispositif 10B l'un de l'autre (figures 10A et 10B).

Afin de protéger les éléments d'étanchéité 40 dans leur état déformé, les capots 70 de protection sont reclippés sur les manchons 60.

On comprend ainsi que l'enserrement des tiges 25 par les éléments d'étanchéité 40 permet d'empêcher l'écoulement du fluide resté prisonnier dans les tubes 100, 200 lors de la déconnexion des dispositifs 10A, 10B.

On comprend également que la déconnexion du premier dispositif 10A du deuxième dispositif 10B n'est possible qu'après la rotation des manchons entraînant le déplacement des corps 30 de la position en saillie à la position affleurante dans laquelle ils sont bloqués par les bras 31.1 des rainures 31, et donc qu'après l'enserrement des tiges 25 par les éléments d'étanchéité 40, ce qui permet d'éviter tout risque d'erreur humaine.

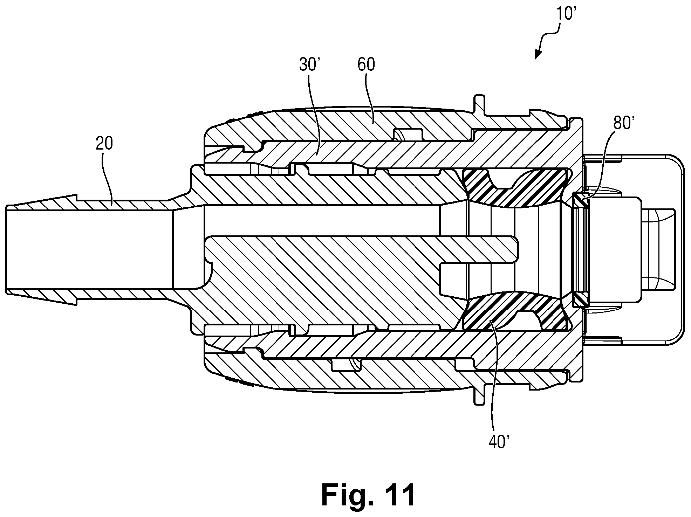

La figure 11 illustre un dispositif 10' de raccordement qui n'est autre qu'une variante du dispositif 10 de raccordement illustré aux figures 1 à 5.

Le dispositif 10' diffère du dispositif 11 en que l'élément d'étanchéité 40' ne s'étend pas en saillie de la face frontale 35' du corps 30' et en ce qu'il comprend un joint d'étanchéité 80' annulaire qui est logé dans un alésage ménagé dans la face frontale 35' du corps 30' et qui s'étend en saillie de ladite face frontale 35' pour assurer l'étanchéité entre le dispositif 10' et un autre dispositif 10'. On comprend que contrairement à l'élément d'étanchéité 40, l'élément d'étanchéité 40' est agencé uniquement pour empêcher la circulation de fluide à travers le dispositif 10' lorsque le corps 30' est en position affleurante, et non pour assurer l'étanchéité entre le dispositif 10' et un autre dispositif 10'.

Bien entendu, l'invention n'est pas limitée au mode de réalisation décrit mais englobe toute variante entrant dans le champ de l'invention telle que définie par les revendications.

Bien que l'ensemble de raccordement comprenne ici deux dispositifs 10A, 10B identiques, il peut aussi comprendre deux dispositifs différents. Par exemple, la patte de verrouillage 36 du premier dispositif 10A peut être remplacée par une broche d'assemblage et la broche d'assemblage 37 du deuxième dispositif 10B peuvent être remplacées par une patte de verrouillage de manière à former un connecteur mâle et un connecteur femelle.

Bien que l'accouplement des dispositifs 10A, 10B soit ici réalisé par clippage, il peut aussi être réalisé par tout autre moyen tel que par vissage d'une partie du dispositif 10A sur l'autre dispositif 10B.

Bien que l'ancrage des tubes 100, 200 sur les dispositifs 10A, 10B soit ici réalisé par un relief en dent de sapin, il peut aussi être réalisé par tout autre moyen tel qu'un jonc, une rondelle extérieurement dentée ou des bras élastiquement déformables.

Bien que le verrouillage du corps 30 en position affleurante soit ici réalisé par l'utilisation de bras 31.1 élastiquement déformable, il peut aussi être réalisé par tout autre moyen tel que par l'utilisation de piges.

Bien que l'élément d'étanchéité 40 soit ici symétrique, il peut être non symétrique.

La tige 25 est optionnelle, notamment lorsque le canal délimité par l'insert 20 est de petit diamètre. L'élément d'étanchéité 40 dans son état déformé obstrue alors en totalité et à lui seul le canal.

Les étapes nécessaires à la déconnexion des dispositifs 10A, 10B peuvent être réalisées simultanément sur chacun des dispositifs 10A, 10B ou bien sur l'un des dispositifs puis l'autre.

## Revendications

1. Dispositif (10) de raccordement aseptique d'un tube, comprenant :
- un insert (20) tubulaire délimitant un canal (21) et ayant un premier tronçon (20.1) d'extrémité pourvu de moyens de sa connexion au tube et un deuxième tronçon (20.2) d'extrémité qui est destiné à s'étendre à l'extérieur du tube ;
- un corps (30) annulaire comprenant un premier tronçon (30.1) d'extrémité monté sur le deuxième tronçon d'extrémité de l'insert et un deuxième tronçon (30.2) d'extrémité s'étendant axialement en saillie du deuxième tronçon d'extrémité de l'insert pour définir avec celui-ci un logement ;
- un élément d'étanchéité (40) annulaire qui est disposé dans le logement entre une face frontale du deuxième tronçon d'extrémité de l'insert et un redan (33) interne du deuxième tronçon du corps annulaire et qui est déformable depuis un premier état dans lequel l'élément d'étanchéité délimite une section de passage de fluide et un deuxième état dans lequel l'élément d'étanchéité obstrue totalement le canal, l'élément d'étanchéité étant agencé pour être amené de son premier état vers son deuxième état sous l'effet d'une compression axiale ; et
- des moyens de couplage (36, 63) à un autre dispositif de raccordement,
le premier tronçon d'extrémité du corps étant monté sur l'insert pour être déplaçable axialement entre une position en saillie axiale de l'insert dans laquelle l'élément d'étanchéité est dans son premier état et une position affleurante de l'insert dans laquelle l'élément d'étanchéité est suffisamment comprimé axialement pour être dans son deuxième état,
**caractérisé en ce que** :
- un manchon (60) de manœuvre est monté mobile en rotation sur le corps entre une position de connexion et une position de déconnexion, et est couplé à l'insert de manière à ce qu'une rotation du manchon de la position de connexion à la position de déconnexion entraîne un déplacement axial du corps de la position en saillie à la position affleurante ; et
- les moyens de couplage sont agencés pour permettre un découplage du dispositif de raccordement et de l'autre dispositif de raccordement uniquement lorsque le manchon est en position de déconnexion.

2. Dispositif selon la revendication 1, comprenant des moyens de verrouillage (29.1, 31.1) du corps (30) en position affleurante.

3. Dispositif selon l'une quelconque des revendications précédentes, dans lequel l'élément d'étanchéité (40) comprend une face frontale en saillie du deuxième tronçon du corps annulaire et sur laquelle est fixé de manière amovible un film de protection (50) sur lequel est rabattue une languette (51) de tirage ayant une première extrémité reliée à un bord du film de protection et une deuxième extrémité s'étendant en saillie d'un bord du film opposé à la première extrémité de la languette de tirage.

4. Dispositif selon l'une quelconque des revendications précédentes, dans lequel le premier tronçon d'extrémité du corps comprend une patte de verrouillage (36) s'étendant en saillie d'une face frontale du corps (30) et comportant à une extrémité libre une aspérité (36.1) destinée à coopérer avec un bord de l'autre dispositif de raccordement, et dans lequel une extrémité du manchon (60) est pourvue extérieurement d'au moins une portion de collet (63) destinée à coopérer avec une patte de verrouillage de l'autre dispositif de raccordement, la portion de collet et la patte de verrouillage formant les moyens de verrouillage.

5. Dispositif selon l'une quelconque des revendications précédentes, dans lequel l'insert tubulaire est pourvu d'une tige (25) s'étendant axialement en porte-à-faux dans le canal pour avoir une portion d'extrémité (25.1) libre en saillie du deuxième tronçon (20.2) d'extrémité de l'insert (20), la section de passage de fluide délimité par l'élément d'étanchéité (40) dans son premier état étant supérieure à une section transversale de la tige, et l'élément d'étanchéité dans son deuxième état enserrant la tige.

6. Dispositif selon l'une quelconque des revendications précédentes, dans lequel la rotation du manchon (60) depuis la position de connexion à la position de déconnexion est sensiblement égale à 180 degrés.

7. Ensemble (1) de raccordement de deux tubes (100, 200) comprenant deux dispositifs (10A, 10B) de raccordement selon l'une quelconque des revendications précédentes.

8. Ensemble (1) selon la revendication 7, dans lequel les deux dispositifs (10A, 10B) de raccordement sont identiques.

9. Procédé de déconnexion de deux tubes (100, 200) raccordés à l'aide d'un ensemble de raccordement selon la revendication 8, le procédé comprenant les étapes suivantes :
- amener les manchons (60) des deux dispositifs de la position de connexion à la position de déconnexion ; et
- éloigner axialement les dispositifs l'un de l'autre.

## Patentansprüche

1. Kupplungsvorrichtung (10) zur aseptischen Verbindung eines Rohres, wobei die Vorrichtung umfasst:
- einen rohrförmigen Einsatz (20), der einen Kanal (21) begrenzt und Folgendes aufweist: einen ersten Endabschnitt (20.1), der mit Mitteln zu dessen Anschluss an das Rohr versehen ist, und einen zweiten Endabschnitt (20.2), der dazu bestimmt ist, sich aus dem Rohr heraus nach außen zu erstrecken;
- einen ringförmigen Körper (30), welcher Folgendes umfasst: einen ersten Endabschnitt (30.1), der auf dem zweiten Endabschnitt des Einsatzes aufmontiert ist, und einen zweiten Endabschnitt (30.2), der sich axial vorstehend von dem zweiten Endabschnitt des Einsatzes erstreckt, um gemeinsam mit diesem eine Aufnahme zu definieren;
- ein ringförmiges Dichtungselement (40), welches in der Aufnahme zwischen einer Stirnfläche des zweiten Endabschnitts des Einsatzes und einer einwärtsgewandten Stufe (33) des zweiten Abschnitts des ringförmigen Körpers angeordnet ist und welches zwischen einem ersten Zustand, in dem das Dichtungselement einen Fluid-Durchgangsquerschnitt begrenzt, und einem zweiten Zustand, in dem das Dichtungselement den Kanal zur Gänze abdichtet, verformbar ist, wobei das Dichtungselement derart angeordnet ist, dass es unter axialer Kompression von seinem ersten Zustand in seinen zweiten Zustand gebracht werden kann; und
- Kupplungsmittel (36, 63) zum Koppeln mit einer anderen Kupplungsvorrichtung,
wobei der erste Endabschnitt des Körpers axial verschiebbar auf dem Einsatz aufmontiert ist, und zwar zwischen einer axial vorstehenden Position des Einsatzes, in welcher sich das Dichtungselement in seinem ersten Zustand befindet, und einer mit dem Einsatz bündig abschließenden Position, in welcher das Dichtungselement in axialer Richtung so weit komprimiert ist, dass es seinen zweiten Zustand einnimmt,
**dadurch gekennzeichnet, dass**
- eine Bedienmuffe (60) drehbeweglich zwischen einer Anschlussposition und einer Nicht-Anschlussposition auf dem Körper montiert ist und derart mit dem Einsatz gekoppelt ist, dass eine Drehung der Muffe von der Anschlussposition in die Nicht-Anschlussposition eine axiale Verschiebung des Körpers von der axial vorstehenden Position in die bündig abschließende Position bewirkt; und
- die Kupplungsmittel dafür ausgelegt sind, ein Entkoppeln der Kupplungsvorrichtung von der anderen Kupplungsvorrichtung nur dann zu ermöglichen, wenn sich die Muffe in der Nicht-Anschlussposition befindet.

2. Vorrichtung nach Anspruch 1, umfassend Verriegelungsmittel (29.1, 31.1) zur Verriegelung des Körpers (30) in der bündig abschließenden Position.

3. Vorrichtung nach einem der vorhergehenden Ansprüche, wobei das Dichtungselement (40) eine Stirnseite umfasst, welche über den zweiten Abschnitt des ringförmigen Körpers hinaus vorsteht und an welcher eine Schutzfolie (50) lösbar befestigt ist, über die eine Ziehlasche (51) umgeschlagen ist, die ein erstes Ende, das mit einem Rand der Schutzfolie verbunden ist, und ein zweites Ende, das sich über einen dem ersten Ende der Ziehlasche entgegengesetzten Rand der Folie hinaus erstreckt, aufweist.

4. Vorrichtung nach einem der vorhergehenden Ansprüche, wobei der erste Endabschnitt des Körpers eine Verriegelungslasche (36) umfasst, die sich über eine Stirnseite des Körpers (30) hinaus erstreckt und an einem freien Ende eine Unebenheit (36.1) enthält, die dazu bestimmt ist, mit einem Rand der anderen Kupplungsvorrichtung zusammenzuwirken, und wobei ein Ende der Muffe (60) außen mit zumindest einem Halsabschnitt (63) versehen ist, der dazu bestimmt ist, mit einer Verriegelungslasche der anderen Kupplungsvorrichtung zusammenzuwirken, wobei der Halsabschnitt und die Verriegelungslasche die Verriegelungsmittel bilden.

5. Vorrichtung nach einem der vorhergehenden Ansprüche, wobei der rohrförmige Einsatz mit einem Schaft (25) versehen ist, welcher sich derart axial freitragend in dem Kanal erstreckt, dass sein freier Endabschnitt (25.1) aus dem zweiten Endabschnitt (20.2) des Einsatzes (20) herausragt, wobei der von dem Dichtungselement (40) in seinem ersten Zustand begrenzte Fluid-Durchgangsquerschnitt größer als ein Querschnitt des Schafts ist, und wobei das Dichtungselement in seinem zweiten Zustand den Schaft abdichtend umschließt.

6. Vorrichtung nach einem der vorhergehenden Ansprüche, wobei die Drehung der Muffe (60) von der Anschlussposition in die Nicht-Anschlussposition im Wesentlichen 180 Grad beträgt.

7. Kupplungsanordnung (1) zur Verbindung zweier Rohre (100, 200), umfassend zwei Kupplungsvorrichtungen (10A, 10B) nach einem der vorhergehenden Ansprüche.

8. Anordnung (1) nach Anspruch 7, wobei die zwei Kupplungsvorrichtungen (10A, 10B) identisch sind.

9. Verfahren zur Entkopplung zweier Rohre (100, 200), die mittels einer Kupplungsanordnung nach Anspruch 8 miteinander verbunden sind, wobei das Verfahren die Schritte umfasst, dass:
- die Muffen (60) beider Vorrichtungen von der Anschlussposition in die Nicht-Anschlussposition gebracht werden; und
- die Vorrichtungen in axialer Richtung voneinander entfernt werden.

## Claims

1. Aseptic junction device (10) for a tube, comprising:
- a tubular insert (20) defining a channel (21) and having a first end segment (20.1) provided with means for connecting it to the tube and a second end segment (20.2) which is intended to extend outside the tube;
- an annular body (30) comprising a first end segment (30.1) mounted on the second end segment of the insert, and also a second end segment (30.2) projecting axially from the second end segment of the insert to define therewith a housing;
- an annular sealing element (40) that is disposed in the housing between a front face of the second end segment of the insert and an internal step (33) of the second segment of the annular body and that is deformable from a first state in which the sealing element delimits a fluid flow section and a second state in which the sealing element completely obstructs the channel, the sealing element being arranged to be brought from its first state to its second state under the effect of an axial compression; and
- means (36, 63) for coupling to another junction device,
the first end segment of the body being mounted on the insert so as to be movable axially between an axially projecting position of the insert and in which the sealing element is in its first state, and a flush position with the insert in which the sealing element is sufficiently axially compressed to be in its second state,
**characterised in that**:
- a maneuvering sleeve (60) is rotatably mounted on the body between a connection position and a disconnection position, and is coupled to the insert so that a rotation of the sleeve from the connection position to the disconnection position leads an axial movement of the body from the projecting position to the flush position; and
- the coupling means are arranged to allow decoupling of the junction device and the other junction device, only when the sleeve is in the disconnection position.

2. Device according to claim 1, including locking means (29.1, 31.1) for locking the body (30) in the flush position.

3. Device according to any one of the preceding claims, wherein the sealing element (40) comprises a front face projecting from the second segment of the annular body, and on which a protective film (50) is removably fastened, the film having a pull-tongue (51) folded down thereon with a first end connected to an edge of the protective film and a second end projecting from the edge of the film that is opposite the first end of the pull-tongue.

4. Device according to any one of the preceding claims, wherein the first end segment of the body comprises a locking tab (36) projecting from a front face of the body (30) and including, at a free end, a serration (36.1) intended to co-operate with an edge of the other junction device, and wherein one end of the sleeve (60) is provided externally with at least one collar portion (63) intended to co-operate with a locking tab of the other junction device, the collar portion and the locking tab forming the locking means.

5. Device according to any one of the preceding claims, wherein the tubular insert is provided with a rod (25) extending axially cantilevered into the channel to have a free end portion (25.1) projecting from the second end section (20.2) of the insert (20), the fluid flow section delimited by the sealing element (40) in its first state being greater than a cross-section of the rod, and the sealing element in its second state enclosing the rod.

6. Device according to any one of the preceding claims, wherein the rotation of the sleeve (60) from the connection position to the disconnection position is substantially equal to 180 degrees.

7. Junction assembly (1) of two tubes (100, 200) comprising two junction devices (10A, 10B) according to any one of the preceding claims.

8. Assembly (1) according to claim 7, wherein the two junction devices (10A, 10B) are identical.

9. Method for disconnecting two tubes (100, 200) joined by means of a junction assembly according to claim 8, the method comprising the following steps:
- bringing the sleeves (60) of the two devices from the connection position to the disconnection position; and
- axially moving the devices away from each other.
